# EUROPEAN PATENT APPLICATION

(11) **EP 1 741 691 A1**
(43) Date of publication of application: **10.01.2007**
(21) Application number: 05014585.3
(22) Date of filing: 06.07.2005
(51) Int. Cl.: C07C 2/82, C07C 2/78, C07C 5/09

(54) **Process for the production of ethylene**

(71) Applicant: Saudi Basic Industries Corporation, Riyadh 11422 (SA)
(72) Inventor: Mamedov, Aggadin, 11551 Riyadh (SA); Al-Wahabi, Saeed, 11551 Riyadh (SA); Lin, Yungyi, 11551 Riyadh (SA); Abdelghani, Mohammed S., 11551 Riyadh (SA); Al-Alwan, Akram, 11551 Riyadh (SA)
(74) Representative: Krijgsman, Willem

(57) **Abstract**

A conventional two-stage pyrolysis scheme is used to convert methane containing feed to produce acetylene as an intermediate product which is immediately in situ hydrogenated into ethylene as the final product by hydrogen transfer medium, preferably supplied by higher alkanes, in one process unit - an integrated reactor comprising multiple reaction zones.

## Description

### FIELD OF THE INVENTION

This invention relates to a process for the production of ethylene, and in particular combining traditional thermal pyrolysis for converting methane containing feed such as natural gas to acetylene with an in situ hydrogenation step to produce ethylene.

### PRIOR ART

Methane pyrolysis to acetylene is well known such as a one step partial oxidation process developed by BASF. The most recent features are covered by US Patents Nos. 5,824,834 and 5,789,644. The general reactor configuration and mechanical design is described in US Patent No. 5,789,644. This design includes three major parts, one is a mixing zone with a special diffuser, the second part is a burner zone followed by a reaction zone, and the final part is a quenching zone using an aqueous coolant or heavy oil. The key innovation of this patent is the use of a perforated plate to cover the burner zone tube for special control purpose. US Patent No. 5,824,834 describes the general process scheme and claims some of the critical feed ratios especially the carbon to oxygen ratio. This ratio is used to control the soot formation which can not be completely or effectively eliminated by this process. The ratio of acetylene to syngas can also be controlled by this process.

Acetylene can also be produced by means of the two stage high temperature pyrolysis (HTP) developed by HOECHST (GB Patents Nos. 921,305 and 958,046). This process comprises two main reaction zones and one quenching zone. The first reaction zone serves as a complete combustion to supply the necessary heat for the second reaction zone namely pyrolysis into which a fresh feed of methane is injected. In the quenching zone a water or heavy oil is used as a coolant. Similarly some amount of carbon will be produced.

The reaction of hydrogenation of acetylene to ethylene over Pd/Al₂O₃ catalysts is also known (US Patent No. 5,847,250). Such a reaction has been widely used to purify the ethylene produced in a steam cracker which is contaminated with acetylene in an amount of typically less than 1.5%. This traditional hydrogenation scheme is not economical for mass production of ethylene from acetylene. Another drawback of using the traditional hydrogenation scheme for this purpose is the oligomerization of acetylene into heavy hydrocarbons, which are the precursors of green oil and coke fragments. Because of these undesirable side reactions, the catalyst is deactivated very fast and needs regeneration steps. In addition, some ethylene may be lost due to consecutive deep hydrogenation reactions. Furthermore, due to the high exothermic heat of reaction, the reactor temperature may run away, thus causing a decline of the catalyst selectivity. Judging from all of the facts and possibilities mentioned above, clearly the vapor phase hydrogenation is difficult to control especially with high acetylene concentration.

On the other hand, the hydrogenation temperature may be controllable in a liquid-phase reactor, through which an adequate volume of liquid solvent is re-circulated continuously to maintain a steady temperature so as to reduce the deactivation rate of the catalysts (US Patents Nos. 4,128,595, 5,059,732 and U.S. Patent Applications Nos. 2005/0048658A1, 2005/0049445A1) while the acetylene is mainly dissolved in the solvent. However the type of solvent can affect the extent of hydrogenation as illustrated in US Patent No. 4,128,595. For this practice, the hydrogenation reaction has been carried out at 116-193°C in the presence of Pd/Al₂O₃ catalyst with an inert paraffinic hydrocarbon solvent. Acetylene conversion was maintained at 99% with 84% selectivity for 9 days. However, if DMF solvent is used instead, the acetylene conversion dropped from 100% to 50% in about 17 hours with 75% selectivity.

Similar liquid phase hydrogenation for acetylene has been described in another US Patent No. 5,059,732 that used gasoline as a liquid medium. This method improved the catalyst life, but it induced the formation of heavy hydrocarbons through the oligomerization of acetylene.

US Patent Applications Nos. 2005/0048658A1 and 2005/0049445A1 disclose the method of acetylene hydrogenation by use of NMP as liquid solvent passing over Pd based catalyst. In these patents the reported concentration of acetylene dissolved in the solvent is about 4.2%. Based on such information this implies that the reactor size will be uneconomically large for the mass production of ethylene. This method could not effectively eliminate the oligomerization of acetylene. Although the catalyst was stable for about 6 days (140 hours), it still needed a regeneration step to sustain its activity. US Patent Application No. 2005/0065392A1 discloses a pyrolysis process for acetylene production followed by a traditional liquid phase hydrogenation scheme downstream. The scheme to be implemented complicates the process design in addition to the limited specific production rate due to the acetylene solubility in the solvent.

Deactivation of the Pd containing catalysts by green oil during acetylene hydrogenation was also described in literature (R.K. Edvinsson, A.M. Holmgren and Said, Irandoust., Ind. Eng. Chem. Res. 1995, 34, 94-100). Authors suggested that a special type of monolith reactor can be used for the gas/liquid/solid acetylene hydrogenation reaction. In that work, acetylene hydrogenation was carried out in the presence of Pd/ α -Al₂O₃ supported on the surface of monolith in the liquid phase at 40°C and 20atm pressure with using 3% C₂H₂ + 28% C₂H₄ + 6% to 11% H₂ + N₂ (balance) mixture. The selectivity to ethylene dropped significantly with the increase of C₂H₂ conversion. At 90% of C₂H₂ conversion, the selectivity of C₂H₂ to ethylene was found to be 60%. Nevertheless, the catalyst was not stable in continuous experiments. After 50 hours, the selectivity to ethylene started to decrease. It was also indicated that the influence of the presence of CO in the gas feed mixture was detrimental to the catalyst rate of hydrogenation where at 2400 ppm of CO the hydrogenation rate stopped completely. The reduction of hydrogenation rate was attributed to CO blocking of some of the hydrogen adsorption sites. Clearly it is a must to have a CO purification step for traditional catalytic hydrogenation.

A similar concept of hydrogen transfer was mentioned and practiced in prior art at quiet different reaction conditions (US Patents Nos. 3,267,170 and 3,321,545) using catalysts. However, the performance pertaining to such a reaction is still constrained by catalyst activity and selectivity. This also requires separate hydrogen transfer reactor downstream and require prior gas purification step.

### SUMMARY OF THE INVENTION

Due to the aforementioned problems resulting from catalyst deactivation, deep hydrogenation, green oil formation, and temperature runaway as well as the low production rate per unit reactor volume, it is necessary to develop a more efficient and stable hydrogenation scheme.

The inventive process for the production of ethylene comprises the following steps:
thermally converting a feed charge containing methane into acetylene as an intermediate, and
in situ hydrogenating the acetylene produced in the first step into ethylene by a non-catalytic hydrogen transfer mechanism.

Preferably, the thermal conversion is a pyrolysis or a partial oxidation process.

In one alternative, the pyrolysis process is a two-stage process, preferably a high temperature pyrolysis (HTP).

In such HTP process, preferably a methane containing feed and oxygen are preheated to 550°C to 650°C and are fed in stoichiometric ratio or with oxygen slightly below stoichiometric ratio and reacted in a combustion zone (stage 1) to form to gases, which has a temperature of from 900°C to 2000°C and a pressure in the range of from 0.5 to 5 atmospheres, and wherein the hot combustion gas is then passed to a pyrolysis zone where methane is then introduced (stage 2) to form acetylene and wherein the pyrolysis zone is maintained at a temperature of 1300°C to 1600°C, wherein the contact time is 3 to 30 milliseconds and the pressure is maintained from 0.5 to 5 atmospheres.

More preferably, in the reaction zone (stage 2), the contact time is 5 to 10 milliseconds and the pressure is maintained at about 2 atmospheres.

In a preferred embodiment, the methane containing feed is fed into the reaction zone (stage 2) after preheating to about 600°C.

In another aspect of the invention, the partial oxidation process is comprised of the preheating the methane containing feed and oxygen to from 600°C to 700°C wherein the oxygen to methane feed ratio is in sub-stoichiometric ratio from 0.5 to 0.7, preferably about 0.62, and the reaction zone is at a temperature of from 1500°C to 1600°C.

In a preferred embodiment, acetylene, before the in situ hydrogenation, is cooled by partial quenching using a coolant.

Such coolant is preferably selected from the group consisting of water, heavy hydrocarbons, natural gas, ethane, methanol and mixtures thereof.

It is also preferred that the final temperature of the partial quench of the acetylene containing gases is between 800°C and 950°C, more preferably between 880°C and 910°C.

The final temperature of the partial quench may be achieved by spray injection of the coolant into the acetylene containing gases.

In a further aspect, the in situ hydrogenation is performed by intimately mixing the acetylene containing effluent from the thermal pyrolysis or partial oxidation zone with an alkane feed to initiate the necessary reaction.

In a preferred embodiment, the alkane feed rate and temperature together with the coolant rate is adjusted to keep the hydrogenation temperature from 800°C to 950°C, more preferably from 800°C to 910°C.

The alkane feed is preferably selected from the group consisting of ethane, propane, butane, iso-butane or mixtures thereof, most preferably ethane.

The alkane feed may be fed to the hydrogenation zone either as a separate feed or mixed with the coolant.

Preferably, the alkane to acetylene molar ratio ranges from 4:1 1 to 0.2:1, more preferably from 1:1 to 0.25:1.

Preferably, the contact time is from 0.01 to 1.0 seconds, more preferably from 0.05 to 0.8 seconds, and the pressure is from 0.5 to 5 atmospheres.

In a preferred embodiment, the hydrogenation reaction zone is not limited to a single partial quench with a coolant but may be extended to multiple partial quench zones to prevent the temperature runaway due to the exothermic heat of hydrogen transfer and hydrogenation.

The final quench may be conducted for stabilizing the product gases containing ethylene at a temperature of from 90°C to 150°C.

The coolant may be water or heavy oil or natural gas.

In one aspect of the invention, the residual acetylene remaining in the final quench product gas together with the unconverted alkane is separated downstream and is recycled back together with the make-up alkane feed to the in situ hydrogenation zone.

In a preferred embodiment, the unconverted methane is separated from the final quench product gas downstream and is recycled back to the primary feed with the oxygen or sent to the fuel gas.

Moreover, it is also preferred that C₃₊ un-saturated and aromatic compounds contained in the final effluent gases are separated downstream.

Most preferably, the methane containing feed is natural gas.

This process proved to be more efficient than other synthesis schemes while simplifying the overall process design. This innovation offers quite an attractive scheme economically for the mass production of ethylene from natural gas via a well-known and proven acetylene route.

In this new process scheme traditional proven thermal methods for making acetylene from natural gas can be used to produce the acetylene as an intermediate product. In this innovative scheme the intermediate product acetylene is immediately hydrogenated by a non-catalytic in-situ hydrogen transfer mechanism. This is preferably achieved by injection of a desirable amount of hydrocarbon such as alkane into the secondary reaction zone and/or quenching zone to produce ethylene as the final product. A non-catalytic hydrogen transfer step is convenient for design and operation of a high temperature reactor. In this way both pyrolysis and hydrogen transfer is performed in one single process unit. The alkane functions as a template-like reagent to initiate the desired reaction to produce the ethylene.

The effluent from the thermal partial oxidation/pyrolysis processes typically containing 10-20% acetylene, 5-30% H₂, 30-40% H₂O and 9-40% CO can be fed to a secondary hydrogenation reaction zone without any further treatment. To have the best process performance the reactor will operate with a contact time of 0.05 to 1.0 seconds, preferably from 0.14 to 0.6 second, at a temperature of 800°C to 1000°C, preferably between 860°C and 910°C, and at a pressure of 0.5 to 5 atmospheres. This can be achieved by using a proper reactor configuration and partial quenching with a coolant and alkane. The preferred molar ratio of alkane to acetylene is between 4:1 and 0.2:1, most preferred between 1:1 and 0.25:1. The achievable conversion of acetylene to ethylene is 85-92%. The ethylene selectivity from combined ethane and acetylene is 60-65%. Most acetylene which is not converted to ethylene is mainly converted back to saturated alkanes and higher alkenes such as methane, ethane, propylene and butylenes instead of decomposing to coke or forming green oil. Based on the commercially established method to produce acetylene (i.e. partial oxidation or pyrolysis), the overall ethylene yield ranges between 17-45% related to methane feed.

In the outlet mixture, the acetylene concentration decreases to 0.8-1.2 mol% which can be safely recycled back to the hydrogenation zone with the alkane feed thus simplifying the overall plant operation.

This process have significant advantages over the prior art, because it does not suffer from temperature runaway and catalyst deactivation resulting from green oil formation or catalyst poisoning by high CO concentration. This significantly reduces operating and capital cost of the plant due to simplification of downstream processing units by removal of unnecessary units such as hydrogenation unit and gas pretreatment units for CO removal.

By the inventive process, carbon formation formed by thermal cracking of higher hydrocarbon species in the outlet gases can be substantially suppressed by in situ hydrogenation.

### SHORT DESCRIPTION OF DRAWINGS

Figure 1 schematically shows an integrated reactor for the thermal pyrolysis coupled with in-situ hydrogenation of the present invention; and
Figure 2 shows a flow diagram of the inventive process for making ethylene from natural gas.

### PREFERRED EMBODIMENTS

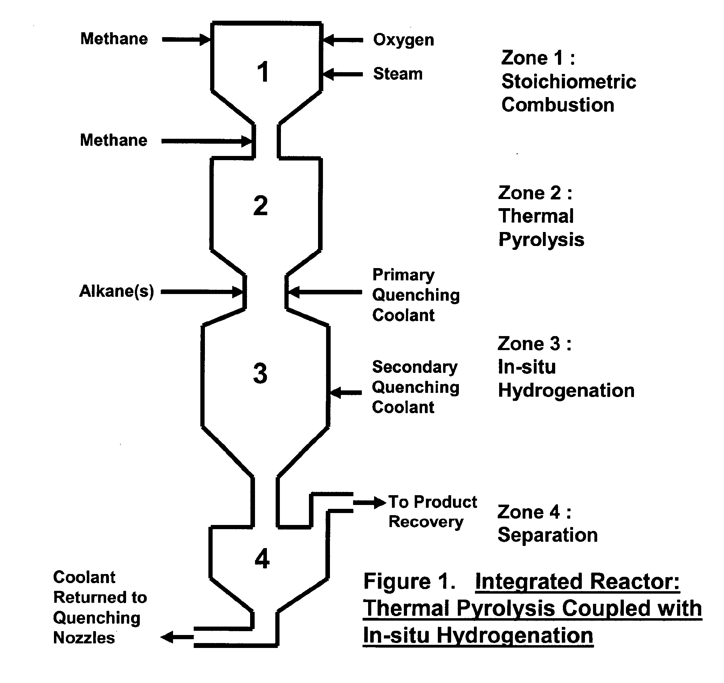

As described above, this combined thermal pyrolysis and in-situ hydrogenation can serve as a compact single unit operation to produce ethylene from natural gas with an economically acceptable yield as shown in Figure 1.

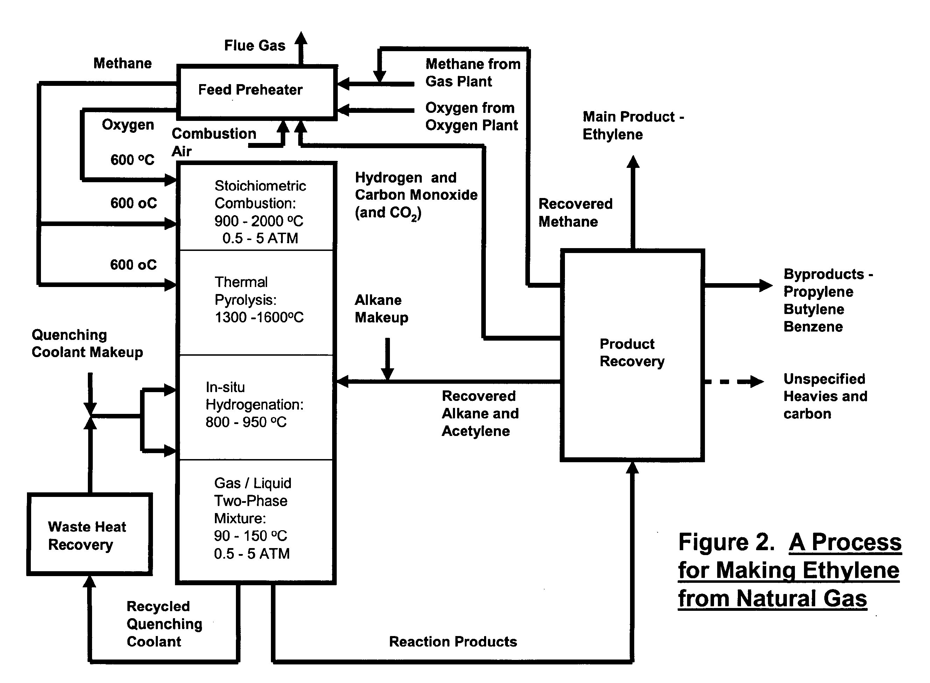

To have a higher conversion of methane per pass, the HOECHST two-stage pyrolysis scheme and condition is selected for this embodiment. The general process scheme is shown in Figure 2 below. Methane obtained from natural gas is combined with unconverted methane recovered from reactor effluent and is preheated to 600°C. An oxygen stream is also preheated before feeding to the reactor. The reactor effluent of CO and hydrogen will be used as a fuel gas for feed preheating and steam generation. The preheated methane is split into two streams one is fed to mixing zone at a stoichiometric ratio with oxygen feed for complete combustion to supply the necessary heat for the endothermic reaction in the pyrolysis zone. The other methane stream is fed to the pyrolysis zone directly.

In the mixing zone the reactor wall can be protected from high temperature by a steam curtain if necessary.

In the mixing zone the temperature ranges between 900°C-2000°C. In the pyrolysis section the preheated methane is quickly mixed with effluents from the mixing zone at 1300°C-1600°C to form acetylene. The desired residence time in the pyrolysis section is controlled in the range of 3 and 30 milliseconds, preferably between 5 and 10 milliseconds. The pressure is set between 0.5-5 atmosphere and preferably 2 atmospheres. Up to this point the traditional two stage pyrolysis is implemented.

To realize the concept of this invention, the pyrolysis products from the pyrolysis zone are partially quenched to the desired hydrogenation reaction temperature of between 800°C to 910°C. The quench can be made with cooling medium mixed alkane. The cooling medium is preferably water, natural gas or methanol but oil can also be used. By doing this most of the acetylene is converted to ethylene by hydrogen transfer. This particular zone is referred to in this disclosure as in-situ hydrogenation zone. The required contact time may vary between 0.05 to 1.0 seconds, preferably from 0.14 -0.6 seconds, and the pressure is set between 0.5-5 atmospheres. The alkane to acetylene ratio can vary from 4:1 1 to 0.2:1. After hydrogenation is complete, the reaction gas is further quenched with a coolant down to 200°C-90°C for product recovery. A two step quench is required for this in-situ hydrogenation operation.

The unconverted acetylene and unconverted alkane are separated and recycled back together to the in-situ hydrogenation zone after being mixed with the fresh feed of alkane and coolant. The remainder gas mixture mainly hydrogen and CO mixed with some CO₂ is used as fuel gas. By using this process scheme there is enough fuel gas to preheat the feed streams to the required temperatures.

In another embodiment a BASF type partial oxidation reactor can be used similarly to make acetylene which is then in-situ hydrogenated in a similar hydrogenation zone to produce ethylene.

Ethylene mass production from natural gas can therefore be economically realized by using this innovative and compact process design.

### EXAMPLES

The pyrolysis and partial oxidation information is available in the prior art, for example, as mentioned herein above. All the necessary process details and information regarding the following in-situ hydrogenation reaction has been tested in the laboratory and verified. All experiments were conducted in a 4ml ID quartz reactor. Typical results are as follows:

### EXAMPLE 1

This example shows the main reaction between acetylene and alkane feed such as ethane at a reaction temperature between 880°C and 890°C. The conversion of acetylene is higher than 90%, while the conversion of ethane is higher than 75%. Additional hydrogen used to hydrogenate the acetylene evidently came from the free hydrogen available in the feed. This is quite different from the Example 2 with no alkane added to the feed.

The flow rate of the feed is controlled to have a constant time. For a typical case when ethane was added to a pyrolysis product gas containing syngas, acetylene and water a feed comprising 8.6% C₂H₂, 4.4% C₂H₆, 5.5% N₂, 13% CO, 68.5% H₂ and water 0.06cc/min was tested. The observed results from the hydrogen transfer reaction are shown in Table 1.

**Table 1**

| Temperature, °C | 880 | 890 |
|---|---|---|
| C₂H₂/C₂H₆ mole ratio | 1.95 | 1.95 |
| C₂H₂ conversion,% | 90.0 | 92.2 |
| C₂H₆ conversion,% | 75.3 | 77.2 |
| Total C₂H₄ selectivity, % | 63.5 | 60.7 |
| Outlet C₂H₂ concentration,% | 1.31 | 1.16 |
| Outlet C₂H₆ concentration,% | 1.12 | 0.95 |
| Outlet C₂H₄ concentration,% | 7.60 | 7.90 |
| Contact time, s | 0.43 | 0.43 |

Additionally it can be seen that high concentration of CO has no effect on acetylene hydrogenation by hydrogen transfer method to the peculiarities of acetylene hydrogenation in contrast to catalytic acetylene hydrogenation on Pd based catalysts. Generally less than 500 ppm level of CO is tolerated in the feed to the catalytic hydrogenation reactor.

### EXAMPLE 2

To achieve the ethane effect of in-situ hydrogenation reaction a separate experiment was conducted. In this example the effect of acetylene hydrogenation in absence of alkane feed is compared to the effect of switching the feed with alkane present in the same experiment. A contact time of 0.44 seconds was maintained. As can be seen with ethane present in the feed the formation of ethylene in column one is more than three times higher than its concentration with no ethane in the feed.

Table 2 also shows the full outlet composition, some higher hydrocarbons are formed as byproducts of the hydrogenation step in the outlet gas. Little traceable carbon is observed in the particular hydrogenation step after many days on stream.

**Table 2**

| | Temperature, °C | | | | | | | | | |
|---|---|---|---|---|---|---|---|---|---|---|
| | | C₂H₄ | C₂H₂ | C₂H₆ | N₂ | H₂ | C₃H₆ | C₄H₈ | C₆H₆ | CH₄ |
| With ethane | | | | | | | | | | |
| Feed | - | - | 8.8 | 17.2 | 36.0 | 37.2 | | | | |
| Run | 858 | 12.75 | 2.13 | 3.47 | 37.1 | 41.4 | 0.07 | 0.31 | 0.47 | 2.19 |

| Without ethane | | | | | | | | | | |
|---|---|---|---|---|---|---|---|---|---|---|
| Feed | - | - | 13.7 | - | 43.9 | 42.5 | | | | |
| Run | 855 | 3.97 | 4.53 | 0.30 | 48.0 | 41.5 | - | 0.20 | 0.43 | 0.86 |

### EXAMPLE 3

Most of experimental results are reproducible and are carried out at different temperatures with almost the same contact time which is applicable to the hydrogenation condition. A gas mixture of 8.79 mol% C₂H₂, 4 mol% N₂, 83.9 mol% H₂, and 3.05 mol% C₂H₆ was used with a flow rate 107 cc/min and with water flow 0.06cc/min. The pressure in the reactor was atmospheric.

Based on the results shown in Table 3 there is an optimum condition to maximize the ethylene concentration in the outlet.

The main reaction product is ethylene and is accompanied by some byproducts in small amounts of CH₄, C₃H₆, C₄H₈ and benzene. Some byproducts such as alkenes and benzene are considered recoverable valuable byproducts.

**Table 3**

| Temperature, °C | 855 | 870 | 901 | 915 | 932 |
|---|---|---|---|---|---|
| C₂H₂/C₂H₆,mole ratio | 0.34 | 0.34 | 0.34 | 0.34 | 0.34 |
| C₂H₂ conversion,% | 79.3 | 84.1 | 88.8 | 89.8 | 92.9 |
| C₂H₆ conversion,% | 56.1 | 65.0 | 59.9 | 66.7 | 64.6 |
| Total C₂H₄ Total C₂H₄ selectivity, % | 59.6 | 54.5 | 59.8 | 57.1 | 52.0 |
| Outlet C₂H₂ concentration,% | 2.1 | 1.67 | 1.14 | 1.05 | 0.74 |
| Outlet C₂H₆ concentration,% | 1.54 | 1.24 | 1.42 | 1.19 | 1.29 |
| Outlet C₂H₄ concentration,% | 6.0 | 6.12 | 6.71 | 6.67 | 6.33 |
| Contact time, s | 0.44 | 0.44 | 0.43 | 0.43 | 0.42 |

### EXAMPLE 4

In this example the effect of contact time on hydrogenation has been evaluated at a constant reaction temperature of 896°C, the results are shown in Table 4. Feed gas composition used was 12.88% C₂H₂, 1.79% C₂H₆, 5.85% N₂, and 79.4% H₂ with the addition of 0.06 ml/min H₂O. The results reveal that the ethylene concentration can be changed by contact time.

**Table 4**

| | | | |
|---|---|---|---|
| Contact time, s | 0.43 | 0.28 | 0.14 |
| C₂H₂ conversion,% | 93,0 | 68.0 | 53.0 |
| C₂H₆ conversion,% | 16.0 | 60.0 | 72.2 |
| Total C₂H₄ selectivity, % | 46.4 | 56.6 | 57.9 |
| Outlet C₂H₂ concentration,% | 1.06 | 4.41 | 6.15 |
| Outlet C₂H₆ concentration, % | 1.18 | 0.54 | 0.52 |
| Outlet C₂H₄ concentration,% | 6.37 | 5.75 | 4.70 |

### EXAMPLE 5

Besides ethane, higher alkanes were also tested such as propane. The result at different temperatures is shown Table 5 and indicates that propane is not as effective as ethane. The feed composition was 12.03% C₂H₂, 14.57% C₃H₈, 36.7% N₂ and 36.65% H₂ with water 0.06 cc/min.

**Table 5**

| Temperature, °C | 683 | 750 | 831 | 841 |
|---|---|---|---|---|
| C₃H₈/C₂H₂,mole ratio | 1.2 | 1.2 | 1.2 | 1.2 |
| C₂H₂ conversion,% | 4.56 | 22.1 | 67.9 | 88.5 |
| C3H conversion,% | 25.4 | 27.6 | 45.8 | 55.1 |
| Total C₂H₄ selectivity, % | 52.3 | 50.1 | 56.6 | 56.5 |
| Outlet C₂H₂ concentration,% | 9.22 | 8.78 | 6.41 | 5.23 |
| Outlet C₃H₈ concentration,% | 14.3 | 11.45 | 4.59 | 1.63 |
| Outlet C₂H₄ concentration,% | 1.99 | 3.3 | 8.56 | 10.97 |
| Contact time, s | 0.52 | 0.49 | 0.45 | 0.45 |

The features disclosed in the foregoing description and in the claims may, both separately and in any combination thereof, be material for realizing the invention in diverse forms thereof.

## Claims

1. A process for the production of ethylene, comprising the following steps:
thermally converting a feed charge containing methane into acetylene as an intermediate, and
in situ hydrogenating the acetylene produced in the first step into ethylene by a non-catalytic hydrogen transfer mechanism.

2. The process according to claim 1, wherein the thermal conversion is a pyrolysis or a partial oxidation process.

3. The process according to claim 2, wherein the pyrolysis process is a two-stage process.

4. The process according to claim 3, wherein the pyrolysis process is a high temperature pyrolysis (HTP).

5. The process according to claim 4, wherein the methane containing feed and oxygen are preheated to 550°C to 650°C and are fed in stoichiometric ratio or with oxygen slightly below stoichiometric ratio and reacted in a combustion zone (stage 1) to form hot gases, which has a temperature of from 900°C to 2000°C and a pressure in the range of from 0.5 to 5 atmospheres, and wherein the hot combustion gas is then passed to a pyrolysis zone where methane is then introduced (stage 2) to form acetylene and wherein the pyrolysis zone is maintained at a temperature of 1300°C to 1600°C, wherein the contact time is 3 to 30 milliseconds and the pressure is maintained from 0.5 to 5 atmospheres.

6. A process according to claim 5, wherein, in the pyrolysis zone (stage 2), the contact time is 5 to 10 milliseconds and the pressure is maintained at about 2 atmospheres.

7. The process according to claim 6, wherein the methane containing feed is fed into the pyrolysis zone (stage 2) after preheating to about 600°C.

8. The process according to claim 2, wherein the partial oxidation process is comprised of the preheating the methane containing feed and oxygen to from 600°C to 700°C wherein the oxygen to methane feed ratio is in sub-stoichiometric ratio from 0.5 to 0.7 and the reaction zone is at a temperature of from 1500°C to 1600°C.

9. The process according to claim 8, wherein the oxygen to methane feed ratio is about 0.62.

10. The process according to any of the preceding claims, wherein acetylene, before the in situ hydrogenation is cooled by partial quenching using a coolant.

11. The process according to claim 10, wherein the coolant is selected from the group consisting of water, heavy hydrocarbons, natural gas, ethane, methanol and mixtures thereof.

12. The process according to claim 10 or 11, wherein the final temperature of the partial quench mixture of the acetylene containing mixture is between 800°C and 950°C.

13. The process according to claim 12, wherein the final temperature of the partial quench of the acetylene containing mixture is between 880°C and 910°C.

14. The process according to any of claims 10 to 13, wherein the final temperature of the partial quench is achieved by spray injection of the coolant into the acetylene containing gases.

15. The process according to any of the preceding claims, wherein the in situ hydrogenation is performed by intimately mixing the acetylene containing effluent from the thermal pyrolysis or partial oxidation zone with an alkane feed.

16. The process according to claim 15, wherein the alkane feed rate and temperature together with the coolant rate is adjusted to keep the hydrogenation temperature from 800°C to 950°C.

17. The process according to claim 16, wherein the alkane feed rate and temperature together with the coolant rate is adjusted to keep the hydrogenation temperature from 800°C to 910°C.

18. The process according to any of claims 15 to 17, wherein the alkane feed is selected from the group consisting of ethane, propane, butane, iso-butane or mixtures thereof.

19. The process according to claim 18, wherein the alkane feed is ethane.

20. The process according to claim 18 or 19, wherein the alkane feed is fed to the hydrogenation zone either as a separate feed or mixed with the coolant.

21. The process according to any of claims 15 to 20, wherein the alkane to acetylene molar ratio ranges from 4:1 to 0.2:1.

22. The process according to claim 21, wherein the alkane to acetylene molar ratio ranges from 1:1 to 0.25:1.

23. The process according to any of claims 15 to 22, wherein the contact time is from 0.01 to 1.0 seconds and the pressure is from 0.5 to 5 atmospheres.

24. The process according to claim 23, wherein the contact time is from 0.05 to 0.8 seconds.

25. The process according to claims 10 to 24, wherein the hydrogenation reaction zone comprises multiple partial quench zones.

26. The process according to any of the claims 15 to 25, wherein a final quench is conducted for stabilizing the product gases containing ethylene at a temperature of from 90°C to 150°C.

27. The process according to any of the claims 10 to 26, where the coolant is water or heavy oil or natural gas.

28. The process according to claim 26, wherein the residual acetylene remaining in the final quench product gas together with the unconverted alkane is separated downstream and is recycled back together with the make-up alkane feed to the in situ hydrogenation zone.

29. The process according to claim 27 or 28, wherein the unconverted methane is separated from the final quench product gas downstream and is recycled back to the primary feed with the oxygen or sent to the fuel gas.

30. The process according to claim 13, wherein C₃₊ un-saturated and aromatic compounds contained in the final effluent gases are separated downstream.

31. The process according to any of the preceding claims, wherein the methane containing feed is natural gas.

## Amended claims

### Amended claims in accordance with Rule 86(2) EPC.

**1.** A process for the production of ethylene, comprising the following steps:
thermally converting a feed charge containing methane into acetylene as an intermediate, wherein the thermal conversion is a pyrolysis or a partial oxidation process, and
in situ hydrogenating the acetylene produced in the first step into ethylene by a non-catalytic hydrogen transfer mechanism, wherein the in situ hydrogenation is performed by intimately mixing the acetylene containing effluent from the thermal pyrolysis or partial oxidation zone with an ethane feed.

**2.** The process according to claim 1, wherein the pyrolysis process is a two-stage process.

**3.** The process according to claim 2, wherein the pyrolysis process is a high temperature pyrolysis (HTP).

**4.** The process according to claim 3, wherein the methane containing feed and oxygen are preheated to 550°C to 650°C and are fed in stoichiometric ratio or with oxygen slightly below stoichiometric ratio and reacted in a combustion zone (stage 1) to form hot gases, which has a temperature of from 900°C to 2000°C and a pressure in the range of from 0.5 to 5 atmospheres, and wherein the hot combustion gas is then passed to a pyrolysis zone where methane is then introduced (stage 2) to form acetylene and wherein the pyrolysis zone is maintained at a temperature of 1300°C to 1600°C, wherein the contact time is 3 to 30 milliseconds and the pressure is maintained from 0.5 to 5 atmospheres.

**5.** A process according to claim 4, wherein, in the pyrolysis zone (stage 2), the contact time is 5 to 10 milliseconds and the pressure is maintained at about 2 atmospheres.

**6.** The process according to claim 5, wherein the methane containing feed is fed into the pyrolysis zone (stage 2) after preheating to about 600°C.

**7.** The process according to claim 1, wherein the partial oxidation process is comprised of the preheating the methane containing feed and oxygen to from 600°C to 700°C wherein the oxygen to methane feed ratio is in sub-stoichiometric ratio from 0.5 to 0.7 and the reaction zone is at a temperature of from 1500°C to 1600°C.

**8.** The process according to claim 7, wherein the oxygen to methane feed ratio is about 0.62.

**9.** The process according to any of the preceding claims, wherein acetylene, before the in situ hydrogenation is cooled by partial quenching using a coolant.

**10.** The process according to claim 9, wherein the coolant is selected from the group consisting of water, heavy hydrocarbons, natural gas, ethane, methanol and mixtures thereof.

**11.** The process according to claim 9 or 10, wherein the final temperature of the partial quench mixture of the acetylene containing mixture is between 800°C and 950°C.

**12.** The process according to claim 11, wherein the final temperature of the partial quench of the acetylene containing mixture is between 880°C and 910°C.

**13.** The process according to any of claims 9 to 12, wherein the final temperature of the partial quench is achieved by spray injection of the coolant into the acetylene containing gases.

**14.** The process according to claim 1, wherein the ethane feed rate and temperature together with the coolant rate is adjusted to keep the hydrogenation temperature from 800°C to 950°C.

**15.** The process according to claim 14, wherein the ethane feed rate and temperature together with the coolant rate is adjusted to keep the hydrogenation temperature from 800°C to 910°C.

**16.** The process according to any of the preceding claims, wherein the ethane feed is fed to the hydrogenation zone either as a separate feed or mixed with the coolant.

**17.** The process according to any of the preceding claims, wherein the ethane to acetylene molar ratio ranges from 4:1 to 0.2:1.

**18.** The process according to claim 17, wherein the ethane to acetylene molar ratio ranges from 1:1 to 0.25:1.

**19.** The process according to any of the preceding claims, wherein the contact time is from 0.01 to 1.0 seconds and the pressure is from 0.5 to 5 atmospheres.

**20.** The process according to claim 19, wherein the contact time is from 0.05 to 0.8 seconds.

**21.** The process according to claims 9 to 20, wherein the hydrogenation reaction zone comprises multiple partial quench zones.

**22.** The process according to any of the preceding claims, wherein a final quench is conducted for stabilizing the product gases containing ethylene at a temperature of from 90°C to 150°C.

**23.** The process according to any of the claims 9 to 22, where the coolant is water or heavy oil or natural gas.

**24.** The process according to claim 22, wherein the residual acetylene remaining in the final quench product gas together with the unconverted alkane is separated downstream and is recycled back together with the make-up alkane feed to the in situ hydrogenation zone.

**25.** The process according to claim 23 or 24, wherein the unconverted methane is separated from the final quench product gas downstream and is recycled back to the primary feed with the oxygen or sent to the fuel gas.

**26.** The process according to claim 12, wherein C₃₊ un-saturated and aromatic compounds contained in the final effluent gases are separated downstream.

**27.** The process according to any of the preceding claims, wherein the methane containing feed is natural gas.
